# EUROPEAN PATENT SPECIFICATION

(11) **EP 1 682 526 B1**
(45) Date of publication and mention of the grant of the patent: **14.01.2009**
(21) Application number: 04791287.8
(22) Date of filing: 22.10.2004
(51) Int. Cl.: C07D 307/87, C07C 255/50, C07F 3/02

(54) **METHOD FOR THE PREPARATION OF CITALOPRAM**
VERFAHREN ZUR HERSTELLUNG VON CITALOPRAM
PROCEDE DE PREPARATION DE CITALOPRAM

(30) Priority: 28.10.2003 EP 03425693
(43) Date of publication of application: 26.07.2006
(73) Proprietor: Adorkem Technology SpA, 24062 Costa Volpino (Bergamo) (IT)
(72) Inventor: COTTICELLI, Giovanni, I-20063 Cernusco sul Naviglio (Milano) (IT); DI LERNIA, Gianluca, I-20139 Milano (IT); MILANESI, Silvia, I-27042 Bressana Bottarone (Pavia) (IT)
(74) Representative: Pistolesi, Roberto
(86) International application number: PCT/EP2004/052626
(87) International publication number: WO 2005/049595

(56) References cited:
- EP-A- 0 171 943
- WO-A-98/19512
- WO-A-02/060886
- WO-A-03/029236
- US-B1- 6 229 026

## Description

The present invention relates to a process for preparation, in just one series of reactions, of 1-[3-(dimethylamino)propyl]-1-(4-fluorophenyl)-1,3-dihydro-5-isobenzofurancarbonitrile and its pharmaceutically acceptable salts.

### BACKGROUND OF THE INVENTION

The mentioned compound, whose formula of structure is here-below reported, 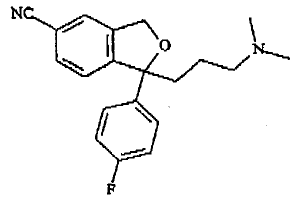 is a well known active drug, better known under its International Denomination "citalopram", which is used in bromhydrate form for the preparation of pharmaceutical compositions for the treatment of depression. Citalopram was first disclosed in the Belgian patent application BE-850401 (and in its equivalent US patent US- 4,136,193); several patent documents also describe methods for its preparation. Preferably, EP-171943 describes a synthetic method with two consecutive Grignard reactions starting from 5-cyanophthalide, the first one with 4-fluorophenylmagnesium bromide, and the second one from the thus-obtained magnesium derivate (formula A), 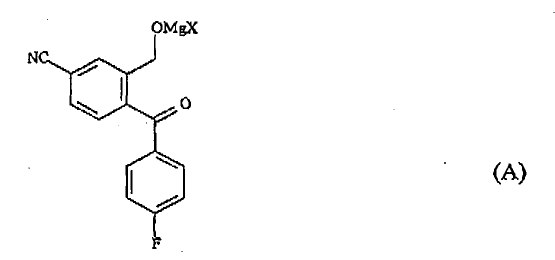 with 3-(dimethylamino)propylmagnesium chloride to obtain first a magnesium intermediate (formula B) 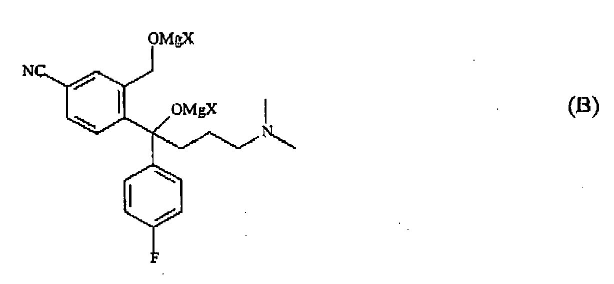 and then, after an acid hydrolysis, a diol (formula C) precursor of citalopram.

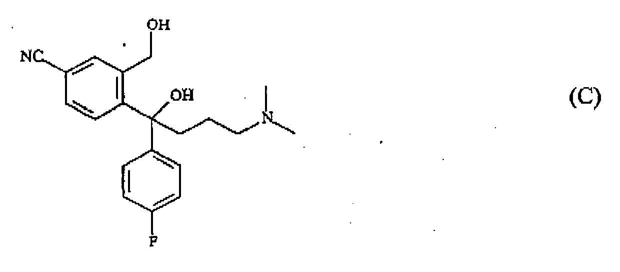

In particular, EP-171943 describes the reaction between 4-fluorophenylmagnesium bromide and 5-cyanophthalide as a slow mechanism so as to state that the intermediate of formula (A) is obtained after a slow transformation from another intermediate (A') as reported here-below:

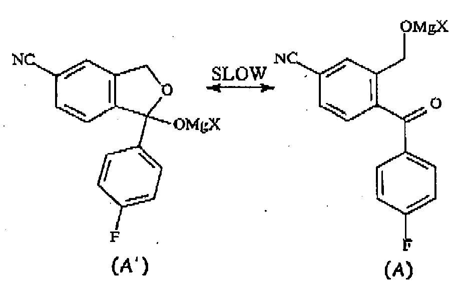

The subsequent reaction between intermediate (A) and 3-(dimethylamino)propylmagnesium chloride is described as slow too (the examples disclose a reaction time of one night).

### SUMMARY OF THE INVENTION

The present invention describes a preparation of citalopram from 5-cyanophthalide by the following route: 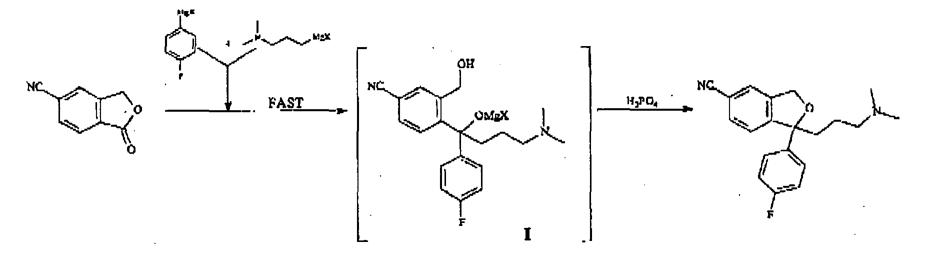 where X is an halogen, preferably chlorine or bromine.

This process is very easy and fast, it doesn't involve the formation of the above-mentioned intermediates (A), (A') and (C) and it allows to obtain citalopram without the isolation of intermediate I.

More in details, intermediate I is synthesized from 5-cyanophthalide by adding a mixture of 4-fluorophenyl magnesium bromide and 3-dimethylaminopropyl magnesium chloride. This reaction, contrarily to EP-171943, is basically instantaneous; besides, at the end of the mixture addition, cyclization is possible by directly adding an acid (preferably orto phosphoric acid 85%) to the reaction mixture, with great operative advantages; after solvent distillation (preferably tetrahydrofuran) the reaction is finished in about two hours.

The subsequent isolation of citalopram is carried out with well known extraction methods.

The present invention relates to a novel method for the preparation of citalopram which comprises:
a) the reaction of 5-cyanophthalide with a mixture of 4-fluorophenyl magnesium halogenide and 3-dimethylaminopropyl magnesium halogenide;
b) the resulting mixture is treated with a phosphine; or with a labile ester forming group, selected from the halide or the anhydride of an organic acid, and a base.

In a preferred embodiment of the invention, 4-fluorophenyl magnesium bromide and 3-dimethylaminopropyl magnesium chloride are employed.

According to a preferred way to proceed, the process according to the invention is carried out "one pot", without isolating the intermediate products until obtainment of citalopram.

The reaction is preferably carried out in an organic polar aprotic solvent, preferably tetrahydrofuran and/or toluene. In practice, the Grignard solution is prepared by adding a solution of 4-fluorobromobenzene in said organic polar aprotic solvent (preferably tetrahydrofuran and/or toluene) to magnesium turnings in presence of traces of iodide at solvent reflux temperature (at 50÷70°C, preferably 70°C for tetrahydrofuran) and cooling to room temperature, after about 30 minutes. Separately a solution of ethylbromide in the same organic solvent is added to magnesium turnings in presence of traces of iodide at solvent reflux temperature; maintaining the mixture at this temperature, 3-dimethylaminopropyl chloride is added to the same solvent, reflux is maintained for further thirty minutes, then it's cooled to room temperature. The two solutions are then mixed together at room temperature.

The mixture is added to a 5-cyanophthalide suspension in the same organic polar aprotic solvent, preferably tetrahydrofuran, at -20÷+20°C, preferably at -10÷0°C. The reaction is usually finished at the end of the addition.

More in general, the ring closure can be achieved with phosphines, preferably with triphenylphosphine. A further possibility is that of reacting the intermediate I with a labile ester forming group selected from the halide or the anhydride of an organic acid and then reacting the so-obtained ester with a base; preferably, the base is selected from triethylamine, dimethylaniline or pyridine. The halide of the organic acid may be that of methanesulfonic, p-toluenesulfonic, trifluoroacetic or trifluoromethanesulfonic acid whereas the halide is preferably the chloride.

In a preferred embodiment of the reaction, from 1.8 to 2.0 moles of 4-fluorophenyl magnesium halogenide are used, preferably about 1.8 moles, and from 1.09 to 1.2 moles of 3-dimethylaminopropyl magnesium halogenide, preferably about 1.1 moles, are used for each mole of 5-cyanophthalide.

According to the best mode to carry out the invention, in order to reduce the production of possible undesired by-products, from 1.7 to 1.6 moles of 4-fluorophenyl magnesium halide, preferably about 1.64, are used for each mole of 3-dimethylaminopropyl magnesium halide; this molar ratio can be for example obtained by mixing 3.4 parts by weight of a 20% 4-fluorophenylmagnesium halide solution in said organic solvent and 1 part by weight of a 30% dimethylaminopropylmagnesium chloride solution in said organic solvent.

Moreover, the reaction is carried out in from 1.0 to 1.6 litres of solvent, preferably about 1.2 litres, for each mole of 5-cyanophthalide.

The citalopram is obtained by extraction preferably with toluene/water firstly in an acid environnement and then in a basic environnement.

On the whole, the invention allows to reduce considerably the reaction and the work-up times, according to a novel and simple way of synthesis. The following examples are for illustraton only and are not intended to limit the invention.

### EXAMPLE 1 (COMPARATIVE)

### 1) 20% solution of 4-fluorophenylmagnesium bromide in tetrahydrofuran

53.5 g of magnesium turnings (2.2 mol) and 0.3 g of iodide are charged into a 4-litres reactor at room temperature under nitrogen. The mixture is then heated to 70°C and a solution of 369.5 g (2.11 mol) of 4-fluorobromobenzene in 1960 ml tetrahydrofuran (5.3 volumes on 4-fluorobromobenzene) is added drop wise, in one hour. After addition the mixture is heated to reflux temperature (68÷70°C) for 30 minutes, then it's cooled to 25°C.

2000g of a 20% solution of 4-fluorophenylmagnesium bromide in tetrahydrofuran are obtained (to be kept under nitrogen and protected from the light).

### 2) 30% solution of dimethylaminopropyl magnesium chloride in tetrahydrofuran

39.22 g (1.61 mol) of magnesium turnings e 0.3 g of iodide are charged into a 2-liters reactor at room temperature under nitrogen. The mixture is then heated to 70°C and a solution of 4.53 ml (0.061 mol) of ethylbromide in 72 ml of tetrahydrofuran is added drop wise in 15 minutes. The reaction is quickly seeded. A solution of 208.77 g (1.90 mol) of dimethylaminopropyl chloride in 545 ml of tetrahydrofuran is added drop wise. After addition the mixture is heated to reflux temperature (68÷70°C) for 30 minutes, then it's cooled to 25°C.

774.1 g of a 30% of dimethylaminopropylmagnesium chloride solution in tetrahydrofuran are obtained (to be kept under nitrogen and protected from the light).

### 3) Citalopram (via orto-phosphoric acid)

A mixture of 1150 g (1.15 mol) of a 20% 4-fluorophenylmagnesium bromide solution and 338 g (0.69 mol) of a 30% dimethylaminopropylmagnesium chloride solution is prepared at room temperature.

The resulting mixture is then added in about 2 hours to a mixture of 100 g (0.63 moles) of 5-cyanophthalide in 750 ml of tetrahydrofuran, under nitrogen at - 0÷0°C. After addition (see note 1), 550 ml of orto-phosphoric acid 85% are added drop wise, keeping the temperature below 10°C. After addition the mixture is heated to 66°C and tetrahydrofuran is distilled; the mixture is then heated to 70÷80°C for 2 hours. The reaction is finished from HPLC control 1100 ml of water and 650 ml of toluen are added.

The phases are separated; the aqueous phase is extracted with 200 ml of toluene to give a new aqueous phase.

9 g of active carbon are added to this new aqueous phase; the mixture is stirred for one hour at 25°C, then it's filtered on a supra panel and washed with water (3x50ml). The filtered is cooled to 5-10°C, 650 ml of toluene are added and about 1300 ml of ammonium hydroxide solution 30% are added till pH 9.5 (keeping the temperature below 15°C). The possible undissolved salts are filtered washing the panel with toluene (about 200 ml) and the phases are separated 300 ml of water are added to the organic phase. The phases are separated. The organic phase is evaporated to crude citalopram as an oil. Crude Citalopram: 177 g

### 4) Citalopram bromhydrate (salyfication)

177 g of crude citalopram obtained from the previous step are dissolved in 300 ml of acetone; it is cooled to 0÷5°C and about 30 ml of hydrobromic acid 62% are added till pH 1. The suspension is stirred at 0÷5°C for one hour and the solvent is then evaporated under vacuum. 200 ml of acetone are added and the solvent is evaporated under vacuum at 40°C; 250 ml of acetone are added and the suspension is stirred at 0÷5°C for a night. The panel is washed with 3x50 ml of acetone at 0÷5°C. The crude citalopram bromhydrate is dried under vacuum at 60°C.
Crude Citalopram bromhydrate: 95,1g

### 5) Citalopram bromhydrate (purification)

95 g of crude citalopram bromhydrate are suspended in 192 ml of deionised water, and the suspension is heated to 60°C to give a solution. 5.7 g of active carbon are added and the mixture is stirred 30 minutes at 60°C. It's filtered on a supra panel at 60°C and the panel is washed with deionised water at 60°C (2x25 ml). The filtered solution is charged in a 500 ml reactor, it's cooled to 0°C and it's stirred for a night. On the following day 140 ml of deionised water are added at 0°C and it's stirred for 5 hours. It's filtered washing the panel with 70 ml of deionised cold water.

The pure citalopram bromhydrate is dried under vacuum at 60°C.

Pure citalopram bromhydrate: 68.8g (molar yield: 27%; w/w yield: 68.8%). NOTE 1: At the end of the addition of the mixture of the two Grignards to the suspension of 5-cyanophthalide, an NMR analysis (BRUKER AMX 3-400) is carried out on a sample.

| ¹H-NMR(DMSO-d6) | | | | |
|---|---|---|---|---|
| δ(ppm) | Multiplicity | N° H | Attribution | |
| 7.83-7.70 | m | 3 | H4, H5, H6 | 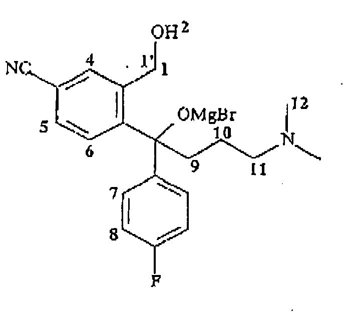 |
| 7.22-7.05 | m | 4 | H7,118 | |
| 5.1 | s | 1 | H2 | |
| 4.52-4.48 | d | 1 | H1 | |
| 4.00-3.96 | d | 1 | H1' | |
| 2.98-2.16 | m | 6 | H9, H10, H11 | |
| 1.69 | s | 6 | H12 | |

This ¹H-NMR is compared to the compound having 2 free hydroxy groups described in EP-171943, here-below reported. By comparison, it's showed that the peak at 6.50 ppm, which corresponds to the hydroxy group in position 3, is lacking in intermediate I.

| ¹H-NMR (DMSO-d6) | | | | |
|---|---|---|---|---|
| δ(ppm) | Multiplicity | N° H | Attribution | |
| 7.89-7.74 | m | 3 | H4, H5, H6 | 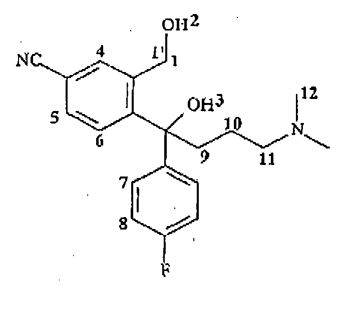 |
| 7.27-7.07 | m | 4 | H7, H8 | |
| 6.50 | s | 1 | H3 | |
| 5.16 | s | 1 | H2 | |
| 4.60-4.56 | d | 1 | H1 | |
| 4.08-4.04 | d | 1 | H1' | |
| 2.29-2.23 | m | 2 | H9 | |
| 2.16-2.11 | m | 2 | H11 | |
| 2.01 | s | 6 | H12 | |
| 1.39-1.19 | m | 2 | H10 | |

## Claims

1. A process for the preparation of citalopram **characterized in that:** (a) 5-cyanophthalide is treated with a mixture of 4-fluorophenyl magnesium halide and 3-dimethylaminopropyl magnesium halide and (b) the obtained mixture is treated with a phosphine, or with a labile ester forming group and a base, wherein the labile ester forming group is selected from the halide or the anhydride of an organic acid.

2. A process according to claim 1, **characterized by** the use of from 1.8 to 2.0 moles of 4-fluorophenyl magnesium halide, preferably about 1.8, for each mole of 5-cyanophthalide.

3. A process according to claim 1, **characterized by** the use of from 1.09 to 1.2 moles of 3-dimethylaminopropyl magnesium halide, preferably about 1.1, for each mole of 5-cyanophthalide.

4. A process according to claim 1, **characterized by** the fact that from 1.7 to 1.6 moles of 4-fluorophenyl magnesium halide, preferably about 1.64, are used for each mole of 3-dimethylaminopropyl magnesium halide.

5. A process according to claims 1-4, **characterized by** the fact that 4-fluorophenyl magnesium halide is a bromine.

6. A process according to claims 1-4, **characterized by** the fact that 3-dimethylaminopropyl magnesium halide is a chloride.

7. A process according to claims 1-6, **characterized in that** the phosphine is thriphenylphosphine.

8. A process according to claim 1, **characterized in that** the halide of the organic acid is the halide of methanesulfonic, p-toluenesulfonic, trifluoroacetic or trifluoromethanesulfonic acid,

9. A process according to claim 8, **characterized in that** the halide is the chloride.

10. A process according to claims 8-9, **characterized in that** base is selected from triethylamine, dimethylaniline or pyridine.

11. A process according to any one of the previous claims, **characterized by** the fact that the process is carried out in an organic polar aprotic solvent.

12. A process according to claim 11, **characterized by** the fact that the process is carried out in from 1.0 to 1.6 litres of solvent, preferably in about 1.2 litres, for each mole of 5-cyanophthalide.

13. A process according to claims 11-12, **characterized by** the fact that the solvent is selected from tetrahydrofuran and/or toluene.

14. A process according to any one of previous claims, **characterized by** the fact that the step (a) is carried out at -20÷+20°C, preferably at -10÷0°C.

15. A process according to any one of previous claims, **characterized by** the fact that the step (b) is carried out at -10÷+20°C, preferably at 0÷+10°C.

16. A process according to any one of previous claims, **characterized by** the fact of being carried out without isolating the intermediate products.

## Patentansprüche

1. Verfahren zur Herstellung von Citalopram, **gekennzeichnet durch**: (a) 5-Cyanophthalid wird mit einer Mischung aus 4-Fluorophenyl Magnesiumhalid und 3-Dimethylaminopropyl Magnesiumhalid behandelt und (b) die erhaltene Mischung wird mit einem Phosphin oder mit einer labilen esterbildenden Gruppe und einer Base behandelt, wobei die labile esterbildenden Gruppe ausgewählt ist aus einem Halid oder einem Anhydrid einer organischen Säure.

2. Verfahren nach Anspruch 1, **gekennzeichnet durch** die Verwendung von 1,8 bis 2,0 Mol, bevorzugt ungefähr 1,8 Mol 4-Fluorophenyl Magnesiumhalid für jedes Mol 5-Cyanophthalid.

3. Verfahren nach Anspruch 1, **gekennzeichnet durch** die Verwendung von 1,09 bis 1,2 Mol, bevorzugt ungefähr 1,1 Mol 3-Dimethylaminopropyl Magnesiumhalid für jedes Mol 5-Cyanophthalid.

4. Verfahren nach Anspruch 1, **dadurch gekennzeichnet, dass** 1,7 bis 1,6 Mol, bevorzugt ungefähr 1,64 Mol 4-Fluorophenyl Magnesiumhalid für jedes Mol 3-Dimethylaminopropyl Magnesiumhalid verwendet werden.

5. Verfahren nach den Ansprüchen 1 bis 4, **dadurch gekennzeichnet, dass** das 4-Fluorophenyl Magnesiumhalid ein Bromid ist.

6. Verfahren nach den Ansprüchen 1 bis 4**, dadurch gekennzeichnet, dass** das 3-Dimethylaminopropyl Magnesiumhalid ein Chlorid ist.

7. Verfahren nach den Ansprüchen 1 bis 6, **dadurch gekennzeichnet, dass** das Phosphin Thriphenylphosphin ist.

8. Verfahren nach Anspruch 1, **dadurch gekennzeichnet, dass** das Halid der organischen Säure das Halid von Methansulfonsäure, p-Toluolsulfonsäure, Trifluoroessigsäure oder Trifluoromethansulfonsäure ist.

9. Verfahren nach Anspruch 8, **dadurch gekennzeichnet, dass** das Halid das Chlorid ist.

10. Verfahren nach Anspruch 8 oder 9, **dadurch gekennzeichnet, dass** die Base aus Triethylamin, Dimethylanilin oder Pyridin ausgewählt ist.

11. Verfahren nach einem der vorhergehenden Ansprüche, **dadurch gekennzeichnet, dass** das Verfahren in einem organischen, polaren, aprotischen Lösungsmittel durchgeführt wird.

12. Verfahren nach Anspruch 11, **dadurch gekennzeichnet, dass** das Verfahren in 1,0 bis 1,6 Liter, bevorzugt in ungefähr 1,2 Liter Lösungsmittel pro Mol 5-Cyanophthalid durchgeführt wird.

13. Verfahren nach den Ansprüchen 11 bis 12, **dadurch gekennzeichnet, dass** das Lösungsmittel aus Tetrahydrofuran und/oder Toluol gewählt ist.

14. Verfahren nach einem der vorhergehenden Ansprüche, **dadurch gekennzeichnet, dass** der Schritt (a) bei -20° C bis +20° C, bevorzugt bei -10° C bis 0° C durchgeführt wird.

15. Verfahren nach einem der vorhergehenden Ansprüche, **dadurch gekennzeichnet, dass** der Schritt (b) bei -10° C bis +20° C, bevorzugt bei 0° C bis + 10° C ausgeführt wird.

16. Verfahren nach einem der vorhergehenden Ansprüche, **dadurch gekennzeichnet, dass** das Verfahren ohne die Isolierung der Zwischenprodukte durchgeführt wird.

## Revendications

1. Procédé de préparation de citalopram **caractérisé en ce que** : (a) du 5-cyanophtalide est traité avec un mélange d'halogénure de 4-fluorophénylmagnésium et d'halogénure de 3-diméthylaminopropyl magnésium et (b) le mélange obtenu est traité avec une phosphine ou avec un groupe formant un ester labile et une base, dans lequel le groupe formant un ester labile est choisi parmi l'halogénure ou l'anhydride d'un acide organique.

2. Procédé selon la revendication 1, **caractérisé par** l'utilisation de 1,8 à 2,0 moles d'halogénure de 4-fluorophénylmagnésium, de préférence environ 1,8, pour chaque mole de 5-cyanophtalide.

3. Procédé selon la revendication 1, **caractérisé par** l'utilisation de 1,09 à 1,2 mole d'halogénure de 3-diméthylaminopropylmagnésium, de préférence environ 1,1, pour chaque mole de 5-cyanophtalide.

4. Procédé selon la revendication 1, **caractérisé par** l'utilisation de 1,7 à 1,6 mole d'halogénure de 4-fluorophénylmagnésium, de préférence environ 1,64, pour chaque mole d'halogénure de 3-diméthylaminopropylmagnésium.

5. Procédé selon les revendications 1 à 4, **caractérisé par le fait que** l'halogénure de 4-fluorophénylmagnésium est un bromure.

6. Procédé selon les revendications 1 à 4, **caractérisé par le fait que** l'halogénure de 3-diméthylaminopropylmagnésium est un chlorure.

7. Procédé selon les revendications 1 à 6, **caractérisé en ce que** la phosphine est la triphénylphosphine.

8. Procédé selon la revendication 1, **caractérisé en ce que** l'halogénure de l'acide organique est l'halogénure de l'acide méthanesulfonique, p-toluènesulfonique, trifluoroacétique ou trifluorométhanesulfonique.

9. Procédé selon la revendication 8, **caractérisé en ce que** l'halogénure est le chlorure.

10. Procédé selon les revendications 8 et 9, **caractérisé en ce que** la base est choisie parmi la triéthylamine, la diméthylaniline ou la pyridine.

11. Procédé selon l'une quelconque des revendications précédentes, **caractérisé par le fait que** le procédé est réalisé dans un solvant aprotique polaire organique.

12. Procédé selon la revendication 11, **caractérisé par le fait que** le procédé est réalisé dans 1,0 à 1,6 litre de solvant, de préférence dans environ 1,2 litre, pour chaque mole de 5-cyanophtalide.

13. Procédé selon les revendications 11 et 12, **caractérisé par le fait que** le solvant est choisi parmi le tétrahydrofurane et/ou le toluène.

14. Procédé selon l'une quelconque des revendications précédentes, **caractérisé par le fait que** l'étape (a) est exécutée à -20-20°C, de préférence à -10-0°C.

15. Procédé selon l'une quelconque des revendications précédentes, **caractérisé par le fait que** l'étape (b) est exécutée à -10-20°C, de préférence à 0-10°C.

16. Procédé selon l'une quelconque des revendications précédentes, **caractérisé par le fait qu'**il est réalisé sans isoler les produits intermédiaires.
